# EUROPÄISCHE PATENTSCHRIFT

(11) **EP 1 473 473 B1**
(45) Veröffentlichungstag und Bekanntmachung des Hinweises auf die Patenterteilung: **27.09.2006**
(21) Anmeldenummer: 03009642.4
(22) Anmeldetag: 29.04.2003
(51) Int. Cl.: F16C 11/10, F16M 11/06

(54) **Feststellbremse und medizinisches Deckenstativ mit einer solchen Feststellbremse**
Blocking device and ceiling mount with such a blocking device
Dispositif de blocage et support de plafond comprenant un tel dispositif

(43) Veröffentlichungstag der Anmeldung: 03.11.2004
(73) Patentinhaber: TRUMPF Kreuzer Medizin Systeme GmbH + Co. KG, 82178 Puchheim (DE)
(72) Erfinder: Bauer, Georg, 85221 Dachau (DE)
(74) Vertreter: Hofer, Dorothea

(56) Entgegenhaltungen:
- EP-A- 0 489 530
- DE-A- 10 000 869
- DE-U- 9 321 260
- US-A- 5 148 467
- US-B1- 6 282 264

## Beschreibung

Die vorliegende Erfindung bezieht sich auf ein medizinisches Deckenstativ mit einer Feststellbremse.

Es sind medizinische Deckenstative bekannt, die zur hängenden Aufnahme von medizinischen Geräten, wie beispielsweise Überwachungsmonitoren, Beatmungssystemen, Spritzenpumpen etc., dienen. Sie werden beispielsweise in Operationssälen oder Intensivräumen etc. für die Unterbringung der für Operationen, die Intensivpflege bzw. Untersuchung eines Patienten notwendigen Systeme verwendet.

Deckenstative bestehen aus mindestens einer vertikalen Säule oder einem horizontalen schwenkbaren Arm mit vertikaler Säule, die über eine Drehverbindung drehbar an der Decke befestigt sind, und einem Geräteträger, auch Stativkopf genannt, in dem die notwendigen Anschlüsse und Geräte untergebracht sind. Durch den drehbar gelagerten Arm läßt sich der Stativkopf in einem gewünschten Radius verschwenken, wodurch der Zugang zum Patienten erleichtert wird oder eine ergonomische Arbeitsposition eingestellt werden kann. Der Stativkopf weist einen Handgriff auf, der dazu dient, den Stativkopf in eine gewünschte Position zu verschwenken.

Der Stativkopf kann an einem höhenverstellbaren Arm angebracht sein und kann dadurch zusätzlich zur Verschwenkbarkeit in seiner Höhenposition verstellt werden, indem der Arm motorisch verstellt wird. Der Hubantrieb wird über ein Bedienelement bzw. einen Schalter betätigt. Das Bedienelement ist in dem Handgriff vorgesehen.

Damit der Stativkopf nach dem Verschwenken in seiner gewünschten Endposition bleibt, sind an den Drehlagern Feststellbremsen vorgesehen. Diese können als Friktionsbremsen ausgeführt sein, wobei hier die Bedienkraft groß sein kann. Es kann aber auch mindestens eine Feststellbremse ansteuerbar vorgesehen sein, die in ihrer Ruhestellung die Drehverbindung blockiert. Die ansteuerbaren Feststellbremsen erleichtern auch das gezielte Bewegen des Stativkopfes, da die Feststellung der Drehverbindungen einzeln gelöst werden können. Wird die Feststellbremse gelöst, läßt sich der Stativkopf per Hand verschwenken. Auch hierfür ist in dem Handgriff ein Bedienelement vorgesehen. Die Feststellbremse wurde bisher als Friktionsbremse konzipiert und pneumatisch betätigt. Entweder wurde die Friktionsbremse als aktive Bremse konzipiert, die nur bei Anliegen von Druckluft funktioniert, oder als Ruhebremse, die in ihrer Ruhestellung die Bremsfunktion ausführt und mit Hilfe von Druckluft gelöst wird. Das Betätigen der Bremse erfolgt durch Schalten des Bedienelements. Wird das Bedienelement betätigt, wird die Feststellbremse gelöst, so daß der Stativkopf verschwenkt werden kann. In der gewünschten Endposition angelangt, wird die Festellbremse wieder festgestellt.

In Bereichen, in denen keine Druckluft zur Verfügung steht, können solche medizinischen Deckenstative mit pneumatisch betätigter Feststellbremse jedoch nicht eingesetzt werden. Auch in Systemen, die an sich keine Druckluft benötigen, wie beispielsweise bei reinen Monitorstativen, ist eine pneumatisch betätigte Feststellbremse nicht rentabel, da der Aufwand bzgl. des Verlegens von Druckluftleitungen ausschließlich für diesen Zweck unrentabel ist. Für die Betätigung sind relativ große Ventile notwendig und der benötigte Bauraum hierfür ist dementsprechend ebenfalls groß und konstruktiv aufwendig.

Alternativ werden magnetisch ansteuerbare Friktionsbremsen eingesetzt, bei denen eine durch eine Feder erzeugte Vorspannkraft eines Bremsstössels auf einen Bremsbelag mit Hilfe eines Hebelsystems und eines Magneten aufgehoben wird, um die Bremse zu lösen. Da die Reibungskraft sehr hoch sein muß, um das Blockieren durch eine Friktionsbremse zu realisieren, ist ein relativ großer und leistungsfähiger Elektromagnet notwendig. Der dazu benötigte Bauraum hierfür ist ebenfalls dementsprechend groß und konstruktiv aufwendig. Außerdem ist aufgrund des notwendigen leistungsfähigen Magneten eine spezielle Ansteüerelektronik notwendig, deren Einsatz aufgrund eines hohen Startstroms aufwendig und teuer ist.

Aus der EP-A-O 489 530 ist eine Bremsenanordnung für eine Drehwelle in einem Nuklearreaktor bekannt. Es handelt sich dabei um eine radiale Bremsenbaugruppe, die eine Drehscheibe und Rotorzähne aufweist. Ferner ist ein nicht drehendes Bremsenbauteil vorgesehen, das einen Bremszahn besitzt. Ein Elektromagnet sowie eine Druckfeder dienen dazu, das Bremsenbauteil gegen die Drehscheibe zu drängen, um die Bremszähne mit dem Zahn in dem Bremsenbauteil in Eingriff zu bringen, wenn der Elektromagnet nicht erregt ist, und die Bremszähne mit dem Zahn in dem Bremsenbauteil außer Eingriff zu bringen, wenn der Elektromagnet erregt wird und das Bremsenbauteil in eine zurückgezogene Position, entgegen der Kraft der Feder, bringt.

Ferner ist aus der US-A-5 148 467 eine Röntgenstrahlenhalteeinrichtung mit einer Bremsenvorrichtung bekannt. Die Bremse besteht aus einem Verriegelungszapfen, der in eine Ausnehmung eingreifen kann. Der Verriegelungszapfen wird dabei über eine Druckfeder in den verriegelten Zustand mit der Drehscheibe vorgespannt. Ein Hebelmechanismus ist über ein Betätigungselement ansteuerbar, so dass der Hebelmechanismus den Verriegelungszapfen entgegen der Vorspannkraft der Feder in eine freie Position zurückzieht. Das Betätigungselement wird über einen Elektromagneten angesteuert.

Es ist daher Aufgabe der vorliegenden Erfindung, eine Feststellbremse bereitzustellen, die kostengünstiger hergestellt werden kann, kleiner baut und ohne Druckluft auskommt.

Diese Aufgabe wird mit einem medizinischen Deckenstativ gemäß Anspruch 1 gelöst. Vorteilhafte Weiterbildungen sind Gegenstand der Unteransprüche.

Durch den Einsatz einer Feststellbremse mit einer Formschlußverbindung, sind nur geringe Kräfte zum Herstellen der Blokkierung notwendig, da die Blockierung nicht mehr über eine Friktionskraft erzeugt wird. Die Energie wird nur noch zum Ineinanderbewegen oder zum Auseinanderbewegen zweier Eingriffselemente benötigt, nicht jedoch zur Herstellung der Blockierung selbst.

Der Einsatz eines Elektromagneten erspart das Verlegen von Druckluftleitungen. Da aus vorgenannten Gründen lediglich die Energie zum Auseinanderbewegen zweier Eingriffselemente benötigt wird, nicht jedoch zur Blockierung oder zum Lösen der Friktionsbremsen, muß der hierzu benötigte Elektromagnet daher in vorteilhafter Weise nicht mehr so leistungsstark sein wie bisher. Vielmehr genügt ein kostengünstiger leichter Elektromagnet, der zudem kleiner baut. Gleiches gilt für das Vorspannelement.

Die Verzahnung kann relativ einfach vorgesehen werden, indem ein Eingriffselement vorzugsweise als Zahnkranz aus verschleißfestem Material ausgebildet ist, während das andere Eingriffselement als Kipphebel vorzugsweise mit einem Zahnsegment mit geringerer Verschleißfestigkeit ausgebildet ist. Vorzugsweise ist das Zahnsegment als auswechselbares Einzelteil ausgebildet. Dann muß bei Verschleiß der Zähne nicht gleich der ganze Kipphebel ausgetauscht werden, sondern nur das verschlissene Zahnsegment.

Ein Verzahnungswinkel von 10° ermöglicht ein Überwinden des Eingriffs beider Verzahnungen, wenn der Elektromagnet nicht funktionsfähig ist bzw. nicht betätigt wird, beispielsweise bei Stromausfall. Unterstützend wirkt außerdem die Verwendung der Messinglegierung CuAl10Ni5Fe4 oder von faserverstärktem Kunststoff für das auswechselbare Zahnsegment, da dieses dann in vorteilhafter Weise eine geringere Verschleißfestigkeit aufweist.

Indem der Kipphebel auf der Achse zwischen zwei Tellerfedern gelagert ist, können Stöße in vorteilhafter Weise besser abgefedert werden.

Indem ein Festanschlag auf dem ersten oder dem zweiten Eingriffselement angebracht ist, kann der Schwenkbereich des Stativs in vorteilhafter Weise auf jeden beliebigen Bereich beschränkt werden.

Indem der Festanschlag als Anschlagreiter mit einem Klemmechanismus ausgebildet ist, kann der Festanschlag in vorteilhafter Weise an jeder beliebigen Stelle im Raster der Verzahnungsteilung auf der Verzahnung des Eingriffselementes festgeklemmt werden.

Indem der Festanschlag elastische Anschlagpuffer aufweist, vorzugsweise aus einem Elastomer, können Stöße bei Erreichen des Festanschlags in vorteilhafter Weise abgefedert werden.

Nachfolgend wird die Erfindung anhand einer derzeit bevorzugten Ausführungsform unter Bezugnahme auf die beigefügten Figuren näher erläutert.

Fig. 1 ist eine perspektivische Ansicht einer Feststellbremse mit einem Teil eines Schwenkarms.

Fig. 2 ist eine perspektivische Ansicht der Feststellbremse ohne Schwenkarm.

Fig. 3 ist eine Seitenansicht wesentlicher Komponenten der Feststellbremse aus Fig. 1.

Fig. 4 ist ein Ausschnitt wesentlicher Komponenten der Feststellbremse aus Fig. 1 in der Draufsicht.

Fig. 5 ist ein Ausschnitt wesentlicher Komponenten einer Feststellbremse gemäß einer alternativen Ausführungsform in der Draufsicht.

Fig. 6 ist eine perspektivische Gesamtdarstellung eines medizinischen Deckenstativs.

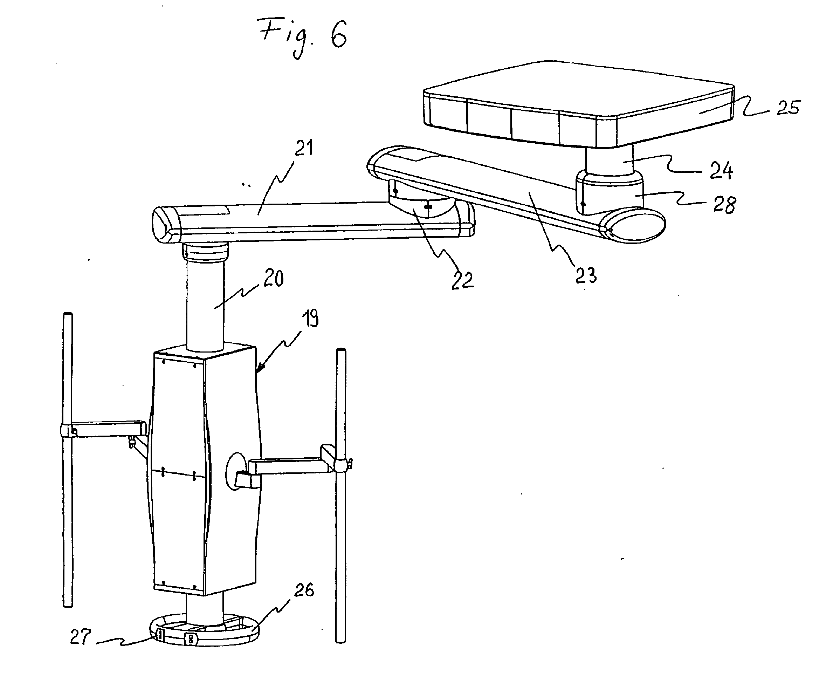

Gemäß Fig. 6 weist ein Deckenstativ einen Stativkopf 19 auf, der an einer vertikalen Säule 20 befestigt ist. Die Säule 20 ist wiederum an einem horizontalen schwenkbaren Arm 21 befestigt. Der Arm 21 ist über eine Drehverbindung 22 drehbar mit einem weiteren horizontalen schwenkbaren Arm 23 verbunden und letzterer ist über eine Drehverbindung 28 und ein kurzes vertikales Säulenstück 24 an einer Deckenaufnahme 25 angebracht. Die Drehverbindung 22 und/oder 28 ist/sind mit einer Feststellbremse ausgestattet, die über Bedienelemente 27, die sich in einem unten am Stativkopf 19 angebrachten Handgriff 26 befinden, gelöst wird.

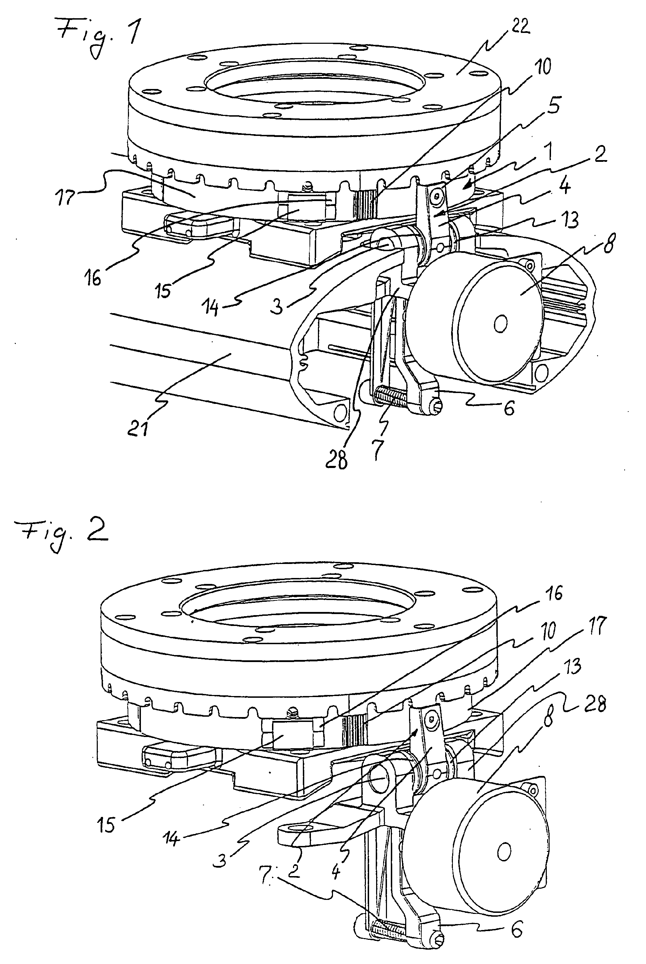

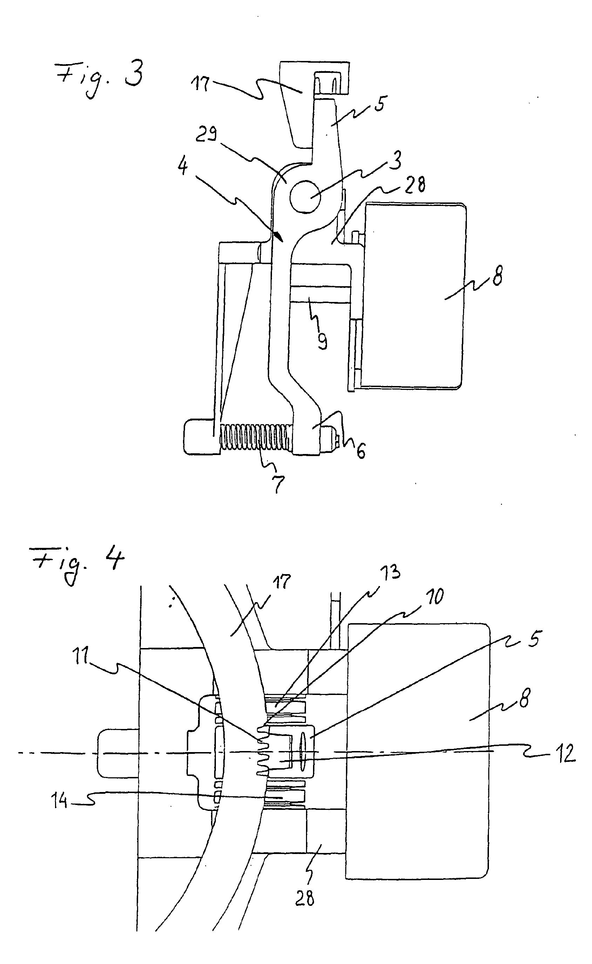

Die Feststellbremse wird im Folgenden unter Bezugnahme auf die Fig. 1 und 2 erläutert. Die Feststellbremse weist ein erstes Eingriffselement 1 auf, das an der Drehverbindung 22 befestigt ist, und ein zweites Eingriffselement 2, das am Arm 21 befestigt ist. Das erste Eingriffselement 1 ist ein Zahnkranz 17, wobei in den Fig. 1 und 2 nur ein kleines Stück einer Verzahnung 10 dargestellt ist. Das zweite Eingriffselement 2 weist einen Kipphebel 4 auf, der über eine Achse 3 an einem am Arm 21 befestigten Träger 28 drehbar gelagert ist.

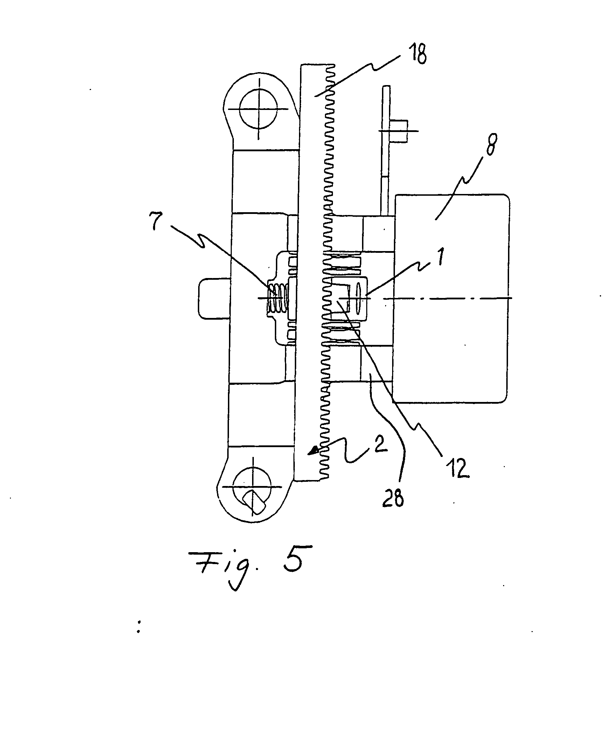

Wie in Fig. 3 am besten zu sehen ist, weist der Kipphebel 4 ein erstes Ende 5 und ein zweites Ende 6 auf. Zwischen den beiden Enden 5 und 6 ist der Kipphebel mit einer Achsaufnahme 29 in Form einer Bohrung ausgestattet. Durch die Achsaufnahme 29 ist eine Achse 3 gesteckt, die drehbar in zwei Lageraugen im Träger 28 gelagert ist. Gemäß Fig. 4 ist am ersten Ende 5 des Kipphebels 4 eine Verzahnung 11 vorgesehen. In der vorliegenden Ausführungsform besteht die Verzahnung aus einem Zahnsegment 12, das drei Zähne aufweist, die in die Verzahnung 10 des Zahnkranzes 17 eingreifen, wenn sich die Feststellbremse in der Bremsposition befindet. In den Fig. 3 und 4 ist die Bremsposition dargestellt. Unter erneuter Bezugnahme auf Fig. 3 ist der Kipphebel 4 an seinem zweiten Ende 6 mit einem Vorspannelement 7 in Form einer Feder so vorgespannt, daß das erste Ende 5 des Kipphebels 4 über die Achse 3 gekippt wird, so daß das erste Ende 5 in Richtung der Verzahnung 10 gedrückt wird und die Verzahnung 11 des Zahnsegments 12 mit den Verzahnung 10 in Eingriff gelangt.

Ferner ist ein Elektromagnet 8 an dem Träger 28 angebracht. Gemäß Fig. 3 weist der Elektromagnet 8 ein Betätigungselement 9 auf, das ein stangenförmiges Ende besitzt, das an dem dem zweiten Ende 6 zugewandten Abschnitt des Kipphebels 4 anliegt. Wird der Elektromagnet 8 erregt, wird das Betätigungselement nach außen gedrückt und schwenkt den Kipphebel 4 im Uhrzeigersinn in Fig. 3 um die Achse 3, so daß die Verzahnung 11 des Zahnsegments 12 außer Eingriff mit der Verzahnung 10 gelangt. Dadurch wird die Feststellbremse gelöst und der Stativkopf 19 kann in die gewünschte Position gedreht werden. Wird der Elektromagnet 8 enterregt, bewegt sich das Betätigungselement 9 wieder zurück und das Vorspannelement 7 drückt die Verzahnung 11 des Zahnsegments 12 wieder in die Verzahnung 10, so daß ein Formschluß zwischen den Verzahnungen 10 und 11 erzeugt wird und die Feststellbremse den Stativkopf 19 wieder blockiert.

Das Zahnsegment 12 besteht aus der relativ weichen Messinglegierung CuA110Ni5Fe4. Alternativ kann auch faserverstarkter Kunststoff verwendet werden. Die Verzahnung 10 des Zahnkranzes 17 dagegen besteht aus relativ verschleißfestem Edelstahl VA (1.4301) nach DIN EN 10027-2. Sollte der Stativkopf trotz festgestellter Bremse, also wenn die Verzahnung 10 mit der Verzahnung 11 in Eingriff steht, mit Gewalt bewegt werden, beispielsweise wenn der Elektromagnet bei Stromausfall nicht mehr erregt werden kann, um die Bremse zu lösen, kann der Formschluß durch einen vorbestimmten Kraftaufwand überwunden werden, da dies durch einen Verzahnungswinkel der Zähne von 10° ermöglicht wird.

In den Fig. 1, 2 und 4 sind Tellerfedern 13, 14 gezeigt, die auf der Achse 3 aufgesteckt sind und sich links und rechts vom Kipphebel 4 befinden. Auf der anderen Seite stützen sie sich an den Lageraugen des Trägers 28 ab. Die Tellerfedern dienen dazu, auf den Stativkopf 19 auftreffende Stöße abzufedern, indem der Kipphebel 4 gegen die Vorspannkraft der Tellerfedern auf der Achse 3 seitlich federnd etwas hin und her bewegt werden kann. Dadurch können beispielsweise heftige Stöße durch ungebremstes in Anschlag fahren, oder durch einen Eingriff der Feststellbremse, bevor das Stativ in Ruhe ist, abgefedert werden.

In Fig. 1 oder 2 ist außerdem ein Festanschlag 15 gezeigt, der am Außenumfang des Zahnkranzes 17 befestigt ist. Die Befestigung erfolgt über einen nicht näher dargestellten Klemmechanismus, mit dem der Festanschlag 15 an jeder beliebigen Position in Abhängigkeit von der Zahnteilung an der Verzahnung 10 des Zahnkranzes befestigt werden kann.

Durch den Festanschlag 15 kann die Bewegungsfreiheit des Stativkopfs 19 den räumlichen Gegebenheiten entsprechend angepaßt werden, indem durch entsprechendes Anbringen zweier Festanschläge 15 beispielsweise ein Winkelbereich von 150° eingestellt wird, wenn nur ein Bewegungsradius von 150° zur Verfügung steht. Um den Stoß noch besser abzudämpfen, wenn der Kipphebel 4 auf den Festanschlag 15 trifft, sind an dessen Seite Anschlagpuffer 16 aus einem Elastomer vorgesehen.

Das Lösen der Feststellbremse erfolgt über die im Handgriff 26 vorgesehenen Bedienelemente 27, die in Fig. 6 dargestellt sind. Wenn die Bedienelemente 27 betätigt werden, wird der Elektromagnet 8 erregt und das Betätigungselement 9 drückt den Kipphebel 4 entgegen der Vorspannkraft der Feder 7 so, daß sich sein erstes Ende 5 weg von der Verzahnung 10 des Zahnkranzes bewegt. Dadurch gelangen die Verzahnungen 10 und 11 außer Eingriff und die Drehverbindung ist gelöst, d.h. der Stativkopf 19 kann in seine gewünschte Position geschwenkt werden. Ist die Zielposition erreicht, wird das Bedienelement wieder losgelassen und der Elektromagnet 8 wird enterregt, so daß der Kipphebel 4 durch die Vorspannkraft der Feder 7 wieder in seine Verriegelungsposition geschwenkt wird, so daß die beiden Verzahnungen 10, 11 wieder in Eingriff gelangen und ein Formschluß hergestellt wird, so daß die Bremse wieder blokkiert.

Gemäß einer alternativen Ausführungsform wie sie in Fig. 5 gezeigt ist, kann die Feststellbremse auch mit einer eine Zahnstange 18 als erstes Eingriffselement verwendet werden, so daß die Relativbewegung zwischen der Zahnstange 18 und dem zweiten Eingriffselement 2 eine Linearbewegung ist.

## Patentansprüche

1. Deckenstativ mit mindestens einer Drehverbindung aus einem ersten Drehelement und einem relativ dazu verdrehbaren zweiten Drehelement eines medizinischen Deckenstativs, mit einer Feststellbremse zur Blockierung einer Relativbewegung eines ersten Elements und eines relativ zum ersten Element versetzbaren zweiten Elements, mit einem am ersten oder zweiten Element angebrachten ersten Eingriffselement (1), einem am anderen Element versetzbar angebrachten zweiten Eingriffselement (2), um mit dem ersten Eingriffselement (1) in oder außer Eingriff zu gelangen, mit einem am anderen Element angreifenden Vorspannelement (7) zum Vorspannen des zweiten Eingriffselements (2) in eine Position, in der das zweite Eingriffselement in eine Eingriffsposition mit dem ersten Eingriffselement gedrängt wird, mit einem Elektromagneten (8) mit einem Betätigungselement (9), das so auf das zweite Eingriffselement (2) wirkt, daß es das zweite Eingriffselement (2) im erregten Zustand des Elektromagneten (8) entgegen der Vorspannkraft des Vorspannelementes (7) in eine Position bringt, in der das zweite Eingriffselement (2) außer Eingriff mit dem erstenEingriffselement (1) bringbar ist, **dadurch gekennzeichnet, daß** das erste und das zweite Eingriffselement (1, 2) eine Verzahnung (10, 11) aufweisen und daß der Verzahnungswinkel der einzelnen Verzahnungen (10, 11) 10° beträgt.

2. Deckenstativ mit einer Feststellbremse nach Anspruch 1, **gekennzeichnet durch** einen um eine Achse (3) am anderen Element drehbar gelagerten Kipphebel (4) mit dem zweiten Eingriffselement (2) an seinem ersten Ende (5), wobei das Vorspannelement (7) zun Vorspannen des Kipphebels (4) in eine Position, in der das zweite Eingriffselement in eine Eingriffsposition mit dem ersten Eingriffselement gedrängt wird, an seinem zweiten Ende (6) angreift.

3. Deckenstativ mit einer Feststellbremse nach Anspruch 1 oder 2, **dadurch gekennzeichnet, daß** das zweite Eingriffselement (2) ein Zahnsegment (12) aus mindestens einem Zahn aufweist, das vorzugsweise auswechselbar ist.

4. Deckenstativ mit einer Feststellbremse nach einem der vorhergehenden Ansprüche , **dadurch gekennzeichnet, daß** eine der Verzahnungen (11) elastisch ausgebildet ist, vorzugsweise aus der Messinglegierung CuAl10Ni5Fe4 oder aus faserverstärktem Kunststoff besteht.

5. Deckenstativ mit einer Feststellbremse nach einem der vorhergehenden Ansprüche , **dadurch gekennzeichnet, daß** eine der Verzahnungen aus Edelstahl VA (1.4301) nach DIN EN 10027-2 besteht.

6. Deckenstativ mit einer Feststellbremse nach einem der vorhergehenden Ansprüche, **dadurch gekennzeichnet, daß** der Kipphebel (4) auf der Achse (3) zwischen zwei Tellerfedern (13, 14) gelagert ist.

7. Deckenstativ mit einer Feststellbremse nach einem der vorhergehenden Ansprüche, **dadurch gekennzeichnet, daß** auf dem ersten oder dem zweiten Eingriffslement ein Festanschlag (15) angebracht ist.

8. Deckenstativ mit einer Feststellbremse nach dem vorhergehenden Anspruch, **dadurch gekennzeichnet, daß** der Festanschlag (15) als Anschlagreiter mit einem Klemmechanismus ausgebildet ist, der im Raster der Verzahnungsteilung auf der Verzahnung festklemmbar ist.

9. Deckenstativ mit einer Feststellbremse nach einem der vorhergehenden Ansprüche 7 oder 8, **dadurch gekennzeichnet, daß** der Festanschlag (15) einen elastischen Anschlagpuffer (16) aufweist

10. Deckenstativ mit einer Feststellbremse nach dem vorhergehenden Anspruch, **dadurch gekennzeichnet, daß** der Anschlagpuffer (16) aus einem Elastomer-Kunststoff besteht.

11. Deckenstativ mit einer Feststellbremse nach einem der vorhergehenden Ansprüche 1 bis 10, **dadurch gekennzeichnet, daß** das erste Eir.griffselement ein Zahnkranz (17) mit Außenverzahnung ist, so daß die Relativbewegung zwischen dem Zahnkranz und dem zweiten Eingriffselement (2) eine Kreisbewegung ist.

12. Deckenstativ mit einer Feststellbremse nach einem der vorhergehenden Ansprüche 1 bis 10, **dadurch gekennzeichnet, daß** das erste Eingriffselement eine Zahnstange (18) ist, so daß die Relativbewegung zwischen der Zahnstange und dem zweiten Eingriffselement eine Linearbewegung ist.

## Claims

1. A ceiling mount with at least one rotary connection formed by a first rotary element and a second rotary element of a medical ceiling mount that can be rotated relatively to the first element, with a locking brake for blocking a relative movement of a first element and of a second element that can be offset relative to the first element, with a first engaging element (1) fitted on the first or second element, a second engaging element (2) fitted displaceably on the other element, in order to engage or disengage with the first engaging element (1), with a pre-tensioning element (7) engaging on the other element for pre-tensioning the second engaging element (2) into a position in which the second engaging element is forced into an engaging position with the first engaging element, with an electromagnet (8) with an actuating element (9), which acts on the second engaging element (2) so that it brings the second engaging element (2), in the excited condition of the electromagnet (8) against the pre-tensioning force of the pre-tensioning element (7), into a position in which the second engaging element (28) can be brought out of engagement with the first engaging element (1), **characterised in that** the first and second engaging element (1,2) have a toothing (10,11) and **in that** the toothing angle of the individual toothings (10,11) is 10°.

2. The ceiling mount with a locking brake according to claim 1, **characterised by** a rocker arm (4) that is pivotably supported about a shaft (3) on the other element, with the second engaging element (2) on its first end (5), wherein the pre-tensioning element (7) engages at its second end (6) for pre-tensioning the rocker arm (4) into a position in which the second engaging element is forced into an engaging position with the first engaging element.

3. The ceiling mount with a locking brake according to claim 1 or 2, **characterised in that** the second engaging element (2) has a toothed segment (12) consisting of at least one tooth which is preferably replaceable.

4. The ceiling mount with a locking brake according to any one of the preceding claims, **characterised in that** one of the toothings (11) is of an elastic design, preferably consisting of the brass alloy CuA110Ni5Fe4 or fibre reinforced plastic.

5. The ceiling mount with a locking brake according to any one of the preceding claims, **characterised in that** one of the toothings consists of high grade steel VA (1.4301) according to DIN EN 10027-2.

6. The ceiling mount with a locking brake according to any one of the preceding claims, **characterised in that** the rocker arm (4) is supported on the shaft (3) between two disk springs (13, 14).

7. The ceiling mount with a locking brake according to any one of the preceding claims, **characterised in that** a fixed stop (15) is installed on the first or second engaging element.

8. The ceiling mount with a locking brake according to any one of the preceding claims, **characterised in that** the fixed stop (15) is designed as a stop slide with a clamping mechanism which can be firmly clamped in the frame of the toothing pitch on the toothing.

9. The ceiling mount with a locking brake according to any one of the preceding claims 7 or 8, **characterised in that** the fixed stop (15) has an elastic stop buffer (16).

10. The ceiling mount with a locking brake according to any one of the preceding claims, **characterised in that** the stop buffer (16) consists of an elastomeric plastic.

11. The ceiling mount with a locking brake according to any one of the preceding claims 1 to 10, **characterised in that** the first engaging element is a toothed rim (17) with outer toothing so that the relative movement between the toothed rim and the second engaging element (2) is a circular movement.

12. The ceiling mount with a locking brake according to any one of the preceding claims 1 to 10, **characterised in that** the first engaging element is a toothed rack (18), so that the relative movement between the toothed rack and the second engaging element is a linear movement.

## Revendications

1. Support en plafond avec au moins un dispositif de liaison rotative, formé d'un premier élément rotatif et d'un deuxième élément rotatif susceptible de tourner par rapport à celui-ci, appartenant à un support en plafond médical, avec un frein de blocage, pour bloquer un déplacement relatif d'un premier élément et d'un deuxième élément déplaçable par rapport au premier élément, avec un premier élément mis en prise (1) monté sur le premier ou le deuxième élément, un deuxième élément de mise en prise (2) monté de façon déplaçable sur l'autre élément, afin de venir en prise ou hors de prise avec le premier élément de mise en prise (1), avec un élément de précontrainte (7), agissant sur l'autre élément pour précontraindre le deuxième élément de mise en prise (2) en une position à laquelle le deuxième élément de mise en prise est forcé en une position de mise en prise avec le premier élément de mise en prise, avec un électroaimant (8) muni d'un élément d'actionnement (9) agissant sur le deuxième élément de mise en prise (2), de manière qu'il mette le deuxième élément de mise en prise (2) lorsque l'électroaimant (8) est excité, à l'encontre d'une force de précontainte de l'élément de précontrainte (7), en une position à laquelle le deuxième élément de mise en prise (2) est susceptible d'être placé hors de prise envers le premier élément de mise en prise (1), **caractérisé en ce que** le premier et le deuxième éléments de mise en prise (1, 2) présentent une denture (10, 11), et **en ce que** l'angle de denture des différentes dentures (10, 11) est de 10°.

2. Support en plafond muni d'un frein de blocage selon la revendication 1, **caractérisé par** un levier de basculement (4) monté en rotation autour d'un axe (3) sur l'autre élément, avec le deuxième élément de mise en prise (2) sur sa première extrémité (5), où l'élément de précontrainte (7), pour la précontrainte du levier de basculement (3), à une position à laquelle le deuxième élément de mise en prise est en une position de mise en prise avec le premier élément de mise en prise, agit sur sa deuxième extrémité (6).

3. Support en plafond avec un frein de blocage selon la revendication 1 ou 2, **caractérisé en ce que** le deuxième élément de mise en prise (2) présente un segment denté (12) formé au moins d'une dent qui, de préférence, est remplaçable.

4. Support en plafond avec un frein de blocage selon l'une des revendications précédentes, **caractérisé en ce que** l'une des dentures (11) est élastique, de préférence et formée de l'alliage de laiton appelé CuAl10Ni5Fe4, ou de matière synthétique renforcée par des fibres.

5. Support en plafond avec un frein de blocage selon l'une des revendications précédentes, **caractérisé en ce que** l'une des dentures est formée d'acier spécial VA (1.4301) selon DIN EN 10027-2.

6. Support en plafond avec un frein de blocage selon l'une des revendications précédentes, **caractérisé en ce que** le levier de basculement (4) et monté sur l'axe (3), entre deux ressorts à disque (13, 14).

7. Support en plafond avec un frein de blocage selon l'une des revendications précédentes, **caractérisé en ce qu'**une butée fixe (15) est montée sur le premier ou le deuxième élément de mise en prise.

8. Support en plafond avec un frein de blocage selon l'une des revendications précédentes, **caractérisé en ce que** la butée fixe (15) et réalisée sous forme de cavalier de butée, avec un mécanisme de serrage, pouvant être bloqué par serrage sur la denture, selon la trame de division de la denture.

9. Support en plafond avec un frein de blocage selon l'une des revendications 7 ou 8 précédentes, **caractérisé en ce que** la butée fixe (15) présente un tampon de butée (16) élastique.

10. Support en plafond avec un frein de blocage selon l'une des revendications précédentes, **caractérisé en ce que** le tampon de butée (16) est formé d'une matière synthétique de type élastomère.

11. Support en plafond avec un frein de blocage selon l'une des revendications 1 à 10 précédentes, **caractérisé en ce que** le premier élément de mise en prise est une couronne dentée (17) munie d'une denture extérieure, de sorte que le mouvement relatif entre la couronne dentée et le deuxième élément de mise en prise (2) est un mouvement circulaire.

12. Support en plafond avec un frein de blocage selon l'une des revendications 1 à 10 précédentes, **caractérisé en ce que** le premier élément de mise en prise est une crémaillère (18), de manière que le déplacement relatif, entre la crémaillère et le deuxième élément de mise en prise, soit un déplacement linéaire.
